# EUROPEAN PATENT APPLICATION

(11) **EP 3 443 901 A1**
(43) Date of publication of application: **20.02.2019**
(21) Application number: 18189212.6
(22) Date of filing: 16.08.2018
(51) Int. Cl.: A61B 5/12

(54) **AUDIOMETRIC TESTING DEVICE**

(30) Priority: 16.08.2017 PL 42257617
(71) Applicant: Centrum Sluchu i Mowy Sp. z o.o., 05-830 Nadarzyn (PL)
(72) Inventor: Bruski, Lukasz, 03-516 Warszawa (PL); Skarzynski, Piotr, 01-494 Warszawa (PL)
(74) Representative: Jedrzejewski, Michal

(57) **Abstract**

The audiometric testing device powered by a battery (13) consisting of a microcontroller (1) connected to a button (15), characterised in that the microcontroller (1) is connected by RAM memory (2), SD card (3), wireless communication module (4) and sound processor (6), where the wireless communication module (4) is also connected to the RF path (5) and the sound processor (6) is connected by a DAC/ADC codec (7), and the battery (13) is connected to the wireless charging system (14).

## Description

The object of the patent is an audiometric testing device. The device is wireless, digital, battery powered, and mobile. The audiometric testing device can be used in audiological tests of various types. Characteristic features of the device are: small size and weight, wireless communication with the control unit, low-level communication protocol, implementation of functionalities required in various types of audiometric tests, wireless data transfer, battery supply and wireless charging, possibility to be used with various types of transducers.

The audiometric testing device powered by a battery consisting of a microcontroller connected to a button, according to the invention is characterised in that the microcontroller is connected by RAM memory, SD card, wireless communication module and sound processor, where the wireless communication module is also connected to the RF path and the sound processor is connected by a DAC/ADC codec, and the battery is connected to the wireless charging system.

Preferably, the DAC/ADC codec is connected to a microphone or air conduction headphones path or a bone conduction headphones path or a loudspeaker path or an input path.

Preferably, the microcontroller is connected to at least one RGB diode.

Small weight and size of the device allow for convenient use in any location, and trouble-free transport. This feature means that the device can be used in professional hearing test chambers, home environments or remote locations (e.g. schools and kindergartens), significantly reducing the time needed to launch the device in a new environment.

The device does not have to be autonomous, it has a set of functionalities required in various audiometric tests run by appropriate commands sent wirelessly from the control unit. The parameters of the commands can be set freely. In contrast to traditional audiometers, the parameter values the parameters are not limited by predefined values, all of the parameter values can be set in the range of 16 bits. This approach, is not available in other solutions, allows for any designs of audiometric test protocols. Depending on the control unit software, the device can be used both as a manual and automatic audiometer. The device may use various sound transducers, however, to guarantee correct operation, it is necessary to calibrate the device with selected transducers.

The device offers the implementation of a low-level communication protocol. This solution, in contrast to traditional audiometers available on the market, allows for a great deal of freedom in the selection of a control unit. The only requirement for control units is the possibility of wireless communication compatible with the device. The result of such an approach is the practical lack of both hardware limitations (the control unit can be: PCs, laptops or netbooks, smartphones, tablets, or dedicated devices based on microcontrollers) and operating system (the device can be operated, among others, by Windows, iOS, Linux, Android). Wireless data transfer allows the user to save audio files transferred from the control unit to the SD card. This solution allows the user to separate yourself from external audio sources such as media players required for traditional audiometers, and change the collection of files used during research without having to physically delete the data carrier.

Battery powered for safe use, without exposure to electric shocks. Wireless battery charging increases the reliability of the device, eliminating the problems resulting from improper connection of the charger connector and connector degradation related to the proper use.

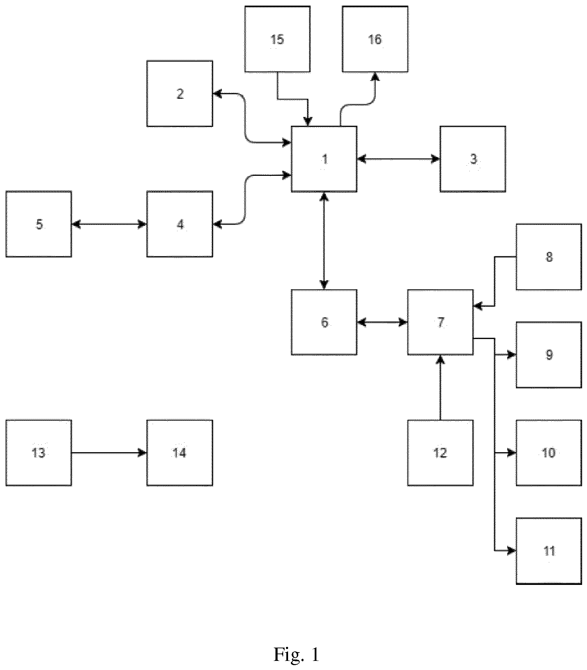

The audiometric testing device is shown in the example in the drawing in which figure 1 shows a block diagram of the device.

### List of signs:

1. Microcontroller
2. RAM memory
3. SD card
4. Wireless communication module
5. RF path
6. Sound processor
7. DAC/ADC codec
8. Microphone
9. Air headphones path
10. Bone headphones path
11. Loudspeakers path
12. Input path
13. Battery
14. Inductive charging system
15. Button
16. RGB diodes

The audiometric testing device is controlled using a microcontroller (1), which is responsible for controlling the operation of the device's logic, battery status (13), and controlling elements such as the wireless communication module (4) and the sound processor (6). The microcontroller (1) is connected by the input with the button (15), which allows to record button presses allowing the examined person to provide relevant information (e.g. in the case of tone audiometry, pressing the button signals hearing the test signal) to the operator. The microcontroller (1) is connected to additional RAM (2) to provide the resources needed to buffer the sound files used during testing. The microcontroller (1) is connected to the SD card (3), the capacity of which must be sufficient to store all sound files used during testing. The sound files can be downloaded to the SD card (3) with the help of the microcontroller (1), without having to remove it from the device. In the example implementation, the STM32F407VGT6 microcontroller was used.

One of the main parts of the device is the sound processor (6). Its tasks include: tone generation, signal shaping, playing sound files, sound correction, correction of signals coming from the microphone (8) and the input path (12) (sent using the codec (7)). The sound processor (6) also allows signals mixing and simultaneous presentation of several signals in different output channels. The sound processor (6) is controlled using the microcontroller (1). The sound processor (6) is connected to the DAC/ADC codec (7), which is used to convert digital signals to analogue signals and convert analogue input signals to digital ones. The codec (7) in the case of clinical tests is additionally connected to at least one of the following elements: air headphones path (9), bone headphones path (10), loudspeakers path (11), input path (12) and the microphone (8). Such a system allows for any redirection of signals to available output paths. In the example implementation, the sound processor (6) uses the ADAU1442 system, while the DAC/ADC codec (7) should have at least a 24-bit resolution and a sampling frequency range of 8-192 kHz. The example implementation uses the codec (7) AD1939.

The wireless communication module (4) is responsible for wireless communication with the control unit connected to the RF path module (5). The wireless communication module (4) enables two-way communication, by means of which orders from the control unit are sent in the form of bit sequences, and to which the device according to the invention responds with the appropriate sequence of bits to confirm receipt of the instruction. The wireless communication module (4) also allows wireless transfer of files used in tests from the control unit to the SD card (3). The wireless communication module (4) also enables sound communication between the examined person and the operator by wireless transmission of the signals coming from the microphone (8). In the example implementation, the CC2564BRVMT module was used.

The device is powered by a battery (13). The battery (13) is charged using the wireless charging system (14). In the exemplary implementation, a charging system compliant with the Qi 1.1 standard has been used, thanks to which cooperation with various induction chargers is possible.

The RGB (16) LEDs are controlled by the microcontroller (1). The use of RGB diodes, compared to monochrome diodes, allows the user to provide more information about the status of the device, e.g. errors in the operation of the device, or battery status through colour coding. Assignment of different colours to each situation allows the operator to recognise many problems in an immediate and unambiguous manner.

Depending on the final purpose of the device, not all blocks must be included. The block diagram shown in the figure refers to the device performing the role of type II (clinical) audiometer. In the event that a type IV (screening) audiometer is sufficient for a given application, the following blocks are not included: microphone (8), bone headphone path (10), loudspeakers path (11) and input path (12).

Such a construction of the device according to the invention allows for unexpected joint acquisition of the following features:
1) Generation and reproduction of pure tones - parameters of generated tones, such as: frequency, sound level, duration, number of repetitions, intervals between repetitions, rise and fall time - can have any values from the available range;
2) Playing audio files from the device memory;
3) Mixing audio signals and the simultaneous presentation of signals in different channels;
4) Correction of sounds;
5) Wireless communication with the control unit through the low-level communication protocol;
6) Easy transportation of the device and easy use in different environmental conditions;
7) Wireless data transfer to device memory;
8) The use of any device that supports wireless communication using the low-level communication protocol as the control unit;
9) The use of different sound transducers.

## Claims

1. The audiometric testing device powered by a battery (13) consisting of a microcontroller (1) connected to a button (15), **characterised in that** the microcontroller (1) is connected by RAM memory (2), SD card (3), wireless communication module (4), and sound processor (6), where the wireless communication module (4) is also connected to the RF path (5) and the sound processor (6) is connected by a DAC/ADC codec (7), and the battery (13) is connected to the wireless charging system (14).

2. The device according to claim 1 **is characterised in that** the DAC/ADC codec (7) is connected to a microphone (8) or air headphones path (9) or a bone headphones path (10) or a loudspeaker path (11) or an input path (12).

3. The device according to claim 1 is **characterised in that** the microcontroller (1) is connected to at least one RGB diode (16).
